# EUROPÄISCHE PATENTSCHRIFT

(11) **EP 0 751 115 B1**
(45) Veröffentlichungstag und Bekanntmachung des Hinweises auf die Patenterteilung: **09.09.1998**
(21) Anmeldenummer: 96109562.7
(22) Anmeldetag: 14.06.1996
(51) Int. Cl.: C07C 201/08, C07C 205/58

(54) **Verfahren zur Herstellung von 5-Fluor-2-nitrobenzoesäure**
Process for the preparation of 5-fluoro-2-nitrobenzoic acid
Procédé pour la préparation de l'acide 5-fluoro-2-nitrobenzoique

(30) Priorität: 27.06.1995 DE 19523244
(43) Veröffentlichungstag der Anmeldung: 02.01.1997
(73) Patentinhaber: BAYER AG, 51368 Leverkusen (DE)
(72) Erfinder: Kissener, Wolfram, Dr., 53819 Neunkirchen-Seelscheid (DE); Kuprat, Jürgen, 42929 Wermelskirchen (DE); Emde, Herbert, Dr., 51069 Köln (DE); Paetz, Klaus-Christian, Dr., 51399 Burscheid (DE)

(56) Entgegenhaltungen:
- EP-A- 0 647 615
- RECUEIL DES TRAVAUX CHIMIQUES DES PAYS-BAS, Bd. 33, 1914, AMSTERDAM NL, Seite 336 XP002012773 SLOTHOUWER:
- TETRAHEDRON LETTERS, Bd. 29, Nr. 40, 1988, OXFORD GB, Seiten 5105-5108, XP000196344 J. DEAN FARMER, JR. ET AL.: "Synthesis and DNA crosslinking ability of a dimeric anthramycin analog"

## Beschreibung

Die vorliegende Erfindung betrifft ein Verfahren zur Herstellung von 5-Fluor-2-nitro-benzoesäure, bei dem ein Produkt erhalten wird, das besonders wenig 3-Fluor-2-nitro-benzoesäure enthält.

5-Fluor-2-nitro-benzoesäure ist ein wichtiges Zwischenprodukt, das nach der Reduktion zur entsprechenden Anthranilsäure zur Herstellung von Pharmazeutikas und Agrochemikalien verwendet wird (siehe z.B. US-PS 3 905 880, US-PS 4 388 472, US-PS 4 824 469, EP-A 109 757, EP-A 183 458, FR-PS 2 541 288 und CZ-PS 159 570). Für diesen Zweck ist es sehr erwünscht, daß man das Zwischenprodukt in möglichst reiner Form zur Verfügung hat, um möglichst reine Wirkstoffe daraus herstellen zu können.

Gemäß dem Stand der Technik (siehe EP-A 647 615) ist es nicht möglich, 3-Fluor-2-nitro-benzoesäure von 5-Fluor-2-nitro-benzoesäure oder deren Reduktionsprodukt 3-Fluoranthranilsäure von 5-Fluoranthranilsäure abzutrennen. Die genannte EP-A schlägt deshalb vor, nicht 3-Fluorbenzoesäure, sondern 3-Fluor-benzoesäureester zu nitrieren und entweder den 3-Fluor-2-nitro-benzoesäureester vom 5-Fluor-2-nitro-benzoesäureester oder nach deren Reduktion den 3-Fluoranthranilsäureester vom 5-Fluoranthranilsäureester durch Destillation oder Schmelzkristallisation abzutrennen.

Um für die Herstellung von Wirkstoffen 5-Fluoranthranilsäure zur Verfügung zu haben muß dann der Ester wieder verseift werden. Dieses Verfahren erfordert hohen Aufwand, da es in jedem Fall die Stufen Veresterung, Isomerentrennung durch Destillation oder Schmelzkristallisation und Esterverseifung umfaßt.

Es besteht deshalb noch das Bedürfnis nach einem weniger aufwendigen Verfahren, mit dem man möglichst reine 5-Fluor-2-nitro-benzoesäure erhalten kann.

Es wurde nun ein Verfahren zur Herstellung von 5-Fluor-2-nitro-benzoesäure durch Nitrierung von 3-Fluorbenzoesäure gefunden, das dadurch gekennzeichnet ist, daß man die Nitrierung in 100 gew.-%iger Schwefelsäure mit beliebigen Gemischen von 100 gew.-%iger Schwefelsäure und 100 gew.-%iger Salpetersäure bei Temperaturen im Bereich von -10 bis +35°C durchführt und 5-Fluor-2-nitro-benzoesäure isoliert und reinigt, indem man, bezogen auf 1 Gew.-Teil eingesetzte 3-Fluorbenzoesäure 7,5 bis 15 Gew.-Teile Wasser vorlegt, in diese Vorlage das ausreagierte Reaktionsgemisch einbringt, den sich bildenden Niederschlag abfiltriert und mit Wasser wäscht.

Auf 1 Gew.-Teil 3-Fluorbenzoesäure kann man beispielsweise 2,5 bis 8, vorzugsweise 3 bis 5 Gew.-Teile 100 gew.-%ige Schwefelsäure einsetzen.

Die genannten Säuregemische können z.B. 20 bis 90 Gew.-% Schwefelsäure und ergänzend zu 100 Gew.-% Salpetersäure enthalten. Vorzugsweise enthalten solche Gemische 50 bis 80 Gew.-% Schwefelsäure und ergänzend zu 100 Gew.-% Salpetersäure. Die Menge der Nitriersäure wird zweckmäßigerweise so bemessen, daß pro Mol 3-Fluorbenzoesäure 1 Mol oder mehr Salpetersäure zum Einsatz gelangt. Vorzugsweise beträgt die Salpetersäuremenge 1 bis 1,5 Mol pro Mol 3-Fluorbenzoesäure.

Wenn zur Herstellung wasserfreier Nitriersäure keine völlig wasserfreie Salpetersäure zur Verfügung steht kann man eine wasserfreie Nitriersäure erhalten, indem man anstelle von wasserfreier Schwefelsäure Oleum oder ein Gemisch aus Oleum und Schwefelsäure einsetzt. Man nimmt dann zweckmäßigerweise Oleum solcher Konzentration bzw. soviel Oleum, daß das darin vorhandene Schwefeltrioxid molmäßig dem Wassergehalt der Salpetersäure entspricht.

Die Nitrierung führt man vorzugsweise bei -5 bis +15°C durch.

Nach Beendigung der Nitrierung erhält man im allgemeinen ein Reaktionsgemisch, das, bezogen auf 5-Fluor-2-nitro-benzoesäure 1 bis 2 Gew.-% 3-Fluor-2-nitro-benzoesäure enthält. Bei relativ höherer Nitriertemperatur, z.B. bei +10 bis +35°C, enthält das Reaktionsgemisch, bezogen auf 5-Fluor-2-nitro-benzoesäure, z.B. 1,5. bis 2,0 Gew.-% 3-Fluor-2-nitro-benzoesäure, bei relativ niedrigerer Nitriertemperatur z.B. bei -10 bis +5°C nur z.B. 1,0 bis 1,5 Gew.-%. Allerdings ist dann die Reaktionszeit länger.

Eine besondere Ausführungsform der erfindungsgemäßen Nitrierung ist dadurch gekennzeichnet, daß das Reaktionsgemisch zu Beginn der Nitrierung zusätzlich wasserentziehende Mittel enthält. Dabei kann es sich beispielsweise um Schwefeltrioxid, Oleum oder Phosphorpentoxid oder um ein Gemisch aus Schwefelsäure. Phosphorsäure und Phosphorpentoxid handeln. Die wasserentziehenden Mittel kann man z.B. dem wasserfreien Nitriermedium und/oder der wasserfreien Nitriersäure zugeben. Bei dieser Ausführungsform der erfindungsgemäßen Nitrierung wird während der Nitrierung entstehendes Reaktionswasser chemisch gebunden. Beispielsweise kann man wasserentziehende Mittel in einer solchen Menge einsetzen, die 10 bis 150 Gew.-% des freiwerdenden Reaktionswassers zu binden vermögen. Vorzugsweise werden soviel wasserentziehende Mittel eingesetzt, wie zur Bindung von 90 bis 110 Gew.-% des freiwerdenden Reaktionswassers benötigt werden.

Beim Arbeiten gemäß dieser Ausführungsform erhält man nach Beendigung der Nitrierung im allgemeinen Reaktionsgemische, die, bezogen auf 5-Fluor-2-nitro-benzoesäure, 0,1 bis 0,5 Gew.-% weniger 3-Fluor-2-nitrobenzoesäure enthalten als bei Durchführung der Nitrierung in Abwesenheit von wasserentziehenden Mitteln.

Nach Beendigung der Nitrierung legt man, bezogen auf 1 Gew.-Teil eingesetzte 3-Fluor-benzoesäure 7,5 bis 15 Gew.-Teile, vorzugsweise 8 bis 12 Gew.-Teile Wasser vor und bringt in diese Vorlage das ausreagierte Reaktionsgemisch ein. Es ist vorteilhaft, hierbei die Temperatur des Gemisches z.B. im Bereich 0 bis +100°C, vorzugsweise im Bereich +30 bis +80°C zu halten. Die Vorlage kann z.B. Frischwasser, Eis und/oder rückgeführtes Waschwasser enthalten. Der sich bildende Niederschlag wird abfiltriert, z.B. bei einer Temperatur im Bereich 10 bis 50°C.

Abschließend wird der abfiltrierte Niederschlag mit Wasser gewaschen. Dadurch wird er von anhaftender Säure befreit und noch vorhandene 3-Fluor-2-nitro-benzoesäure weitgehend herausgelöst. Da sich nicht nur 3-Fluor-2-nitro-benzoesäure, sondern auch 5-Fluor-2-nitro-benzoesäure im Waschwasser löst, ist es zweckmäßig, die für den gegebenen Einzelfall optimale Menge an gesamtem Waschwasser durch einfache Vorversuche zu ermitteln. Dabei gilt, 5-Fluor-2-nitro-benzoesäure wird mit zunehmender Reinheit und mit abnehmender Ausbeute erhalten, wenn man größere Mengen Waschwasser einsetzt.

Im allgemeinen werden befriedigende Ergebnisse erhalten, wenn man, bezogen auf 1 Gew.-Teil eingesetzte 3-Fluor-benzoesäure insgesamt 5 bis 10, vorzugsweise 6 bis 9 Gew.-Teile Waschwasser einsetzt. Es ist vorteilhaft, diese Wäsche mehrstufig zu gestalten, z.B. 2 bis 5-stufig, wobei in den einzelnen Stufen gleiche oder unterschiedliche Mengen an Waschwasser eingesetzt werden können.

Vorteilhafterweise führt man das Waschwasser zurück, d.h. man verwendet es zur Bereitstellung der Vorlage, in die man das ausreagierte Nitriergemisch einbringt.

Das erfindungsgemäße Verfahren gestattet die Herstellung von 5-Fluor-2-nitro-benzoesäure mit Gehalten an 3-Fluor-2-nitro-benzoesäure von im allgemeinen weniger als 0,4 Gew.-%, häufig weniger als 0,2 Gew.-%, wobei zur Reinigung im Gegensatz zum Stand der Technik keine Uberführung in eine andere Verbindungsklasse (Ester statt Säure) und keine aufwendigen Trennoperationen (wie Destillation oder Schmelzkristallisation) nötig sind.

Das erfindungsgemäße Verfahren liefert 5-Fluor-2-nitro-benzoesäure in Ausbeuten von im allgemeinen über 85 % der Theorie. Bei Rückführung des Waschwassers in die Vorlage, in die das ausreagierte Nitriergemisch eingebracht wird, werden im allgemeinen Ausbeuten von über 90 % der Theorie erreicht.

### Beispiele

Die Angaben Gew.-% Fehlisomere beziehen sich stets auf vorhandene 5-Fluor-2-nitro-benzoesäure. Fehlisomere sind im wesentlichen 3-Fluor-2-nitro-benzoesäure.

### Beispiel 1

841 g 3-Fluorbenzoesäure wurden in 3261 g 100 gew.-%iger Schwefelsäure vorgelegt und das Gemisch auf +10°C abgekühlt. Danach wurden 1147 g Nitriersäure bestehend aus 67 Gew.-% 100 gew.-%iger Schwefelsäure und 33 Gew.-% 100 gew.-%iger Salpetersäure zugefügt. Nach Beendigung der Reaktion enthielt das Gemisch ca. 1,65 Gew.-% Fehlisomere. Das Gemisch wurde auf 10 l Wasser gegeben und anschließend auf Raumtemperatur abgekühlt. Das dann ausgefallene Festprodukt wurde abfiltriert und anschließend 3 mal mit je 2 l Wasser gewaschen und getrocknet. Es wurden 957,8 g 5-Fluor-2-nitro-benzoesäure (gerechnet als 100 %ige Ware) erhalten, was 86,3 % der Theorie entspricht. Das Produkt enthielt 0,17 Gew.-% 3-Fluor-2-nitro-benzoesäure.

### Beispiel 2

Es wurde verfahren wie in Beispiel 1, jedoch wurde die Nitrierung bei anderen Temperaturen durchgeführt und das Nitriergemisch nicht weiter aufgearbeitet.
a) Bei einer Nitriertemperatur von +25°C enthielt das Gemisch ca. 1,95 Gew.-% Fehlisomere.
b) Bei einer Nitriertemperatur von -5°C enthielt das Gemisch ca. 1,05 Gew.-% Fehlisomere.

### Beispiel 3

Es wurde verfahren wie in Beispiel 1, jedoch wurde die Nitrierung in Gegenwart wasserentziehender Mittel durchgeführt und das Nitriergemisch nicht weiter aufgearbeitet.
a) Bei Zusatz der zur Bindung des Reaktionswassers theoretisch erforderlichen Menge SO₃ enthielt das Gemisch ca. 1,45 Gew.-% Fehlisomere.
b) Bei Zusatz der zur Bindung des Reaktionswassers theoretisch erforderlichen Menge P₂O₅ enthielt das Gemisch ca. 1,2 Gew.-% Fehlisomere.

### Beispiel 4

Es wurde verfahren wie in Beispiel 1, jedoch wurde bei -5°C gearbeitet und die zur Bindung des Reaktionswassers theoretisch erforderliche Menge SO₃ hinzugefügt. Nach Beendigung der Reaktion enthielt das Gemisch ca. 0,8 % Fehlisomere.

## Patentansprüche

1. Verfahren zur Herstellung von 5-Fluor-2-nitro-benzoesäure durch Nitrierung von 3-Fluorbenzoesäure, dadurch gekennzeichnet, daß man die Nitrierung in 100 gew.-%iger Schwefelsäure mit beliebigen Gemischen von 100 gew.-%iger Schwefelsäure und 100 gew.-%iger Salpetersäure bei Temperaturen im Bereich -10 bis +35°C durchführt und 5-Fluor-2-nitro-benzoesäure isoliert und reinigt, indem man, bezogen auf 1 Gew.-Teil eingesetzte 3-Fluorbenzoesäure 7,5 bis 15 Gew.-Teile Wasser vorlegt, in diese Vorlage das ausreagierte Reaktionsgemisch einbringt, den sich bildenden Niederschlag abfiltriert und mit Wasser wäscht.

2. Verfahren nach Anspruch 1, dadurch gekennzeichnet, daß man eine Nitriersäure einsetzt, die 20 bis 90 Gew.-% 100 %ige Schwefelsäure und ergänzend zu 100 Gew.-% 100 %ige Salpetersäure enthält.

3. Verfahren nach Ansprüchen 1 und 2, dadurch gekennzeichnet, daß man soviel Nitriersäure einsetzt, daß die darin enthaltene Salpetersäure 1 bis 1,5 Mol pro Mol 3-Fluorbenzoesäure beträgt.

4. Verfahren nach Ansprüchen 1 bis 3, dadurch gekennzeichnet, daß man die Nitrierung in zusätzlicher Gegenwart von wasserentziehenden Mitteln durchführt.

5. Verfahren nach Ansprüchen 1 bis 4, dadurch gekennzeichnet, daß man auf 1 Gew.-Teil eingesetzte 3-Fluorbenzoesäure 8 bis 12 Gew.-Teile Wasser vorlegt und beim Einbringen des ausreagierten Reaktionsgemisches in die Vorlage die Temperatur im Bereich 0 bis +100°C hält.

6. Verfahren nach Ansprüchen 1 bis 5, dadurch gekennzeichnet, daß man die Filtration bei einer Temperatur im Bereich +10 bis +50°C durchführt.

7. Verfahren nach Ansprüchen 1 bis 6, dadurch gekennzeichnet, daß man für die Wäsche mit Wasser auf 1 Gew.-Teil eingesetzte 3-Fluorbenzoesäure insgesamt 5 bis 10 Gew.-Teile Waschwasser einsetzt und die Wäsche mehrstufig gestaltet.

8. Verfahren nach Ansprüchen 1 bis 7, dadurch gekennzeichnet, daß man das Waschwasser in die Vorlage zurückführt, in die das ausreagierte Reaktionsgemisch eingebracht wird.

## Claims

1. Process for the preparation of 5-fluoro-2-nitro-benzoic acid by nitration of 3-fluorobenzoic acid, characterized in that the nitration is carried out in 100% strength by weight sulphuric acid with any mixture of 100% strength by weight sulphuric acid and 100% strength by weight nitric acid at temperatures in the range -10 to +35°C and 5-fluoro-2-nitro-benzoic acid is isolated and purified, by taking 7.5 to 15 parts by weight of water, based on one part by weight of 3-fluorobenzoic acid used, introducing the reaction mixture reacted to exhaustion into this volume of water, filtering off the precipitate formed and washing it with water.

2. Process according to Claim 1, characterized in that a nitrating acid is used which contains 20 to 90% by weight of 100% strength sulphuric acid and 100% strength nitric acid to make up 100% by weight.

3. Process according to Claims 1 and 2, characterized in that the amount of nitrating acid used is such that the nitric acid contained therein is 1 to 1.5 mol per mole of 3-fluorobenzoic acid.

4. Process according to Claims 1 to 3, characterized in that the nitration is carried out in the additional presence of dehydrating agents.

5. Process according to Claims 1 to 4, characterized in that, per 1 part by weight of 3-fluorobenzoic acid used, 8 to 12 parts by weight of water are taken and, when the reaction mixture reacted to exhaustion is introduced into the volume of water taken, the temperature is kept in the range 0 to +100°C.

6. Process according to Claims 1 to 5, characterized in that the filtration is carried out at a temperature in the range +10 to +50°C.

7. Process according to Claims 1 to 6, characterized in that, for the washing with water, in total 5 to 10 parts by weight of washing water are used per 1 part by weight of 3-fluorobenzoic acid used and the washing is arranged to be multistage.

8. Process according to Claims 1 to 7, characterized in that the washing water is recycled into the volume taken into which the reaction mixture reacted to exhaustion is introduced.

## Revendications

1. Procédé de préparation de l'acide 5-fluoro-2-nitrobenzoïque par nitration de l'acide 3-fluorobenzoïque, caractérisé en ce que l'on effectue la nitration dans de l'acide sulfurique à 100 % en masse avec des mélanges quelconques d'acide sulfurique à 100 % en masse et d'acide nitrique à 100 % en masse à des températures comprises entre -10°C et +35°C, et on isole et purifie l'acide 5-fluoro-2-nitrobenzoïque par un procédé selon lequel on dispose dans un récipient 7,5 à 15 parties en masse d'eau pour 1 partie en masse d'acide 3-fluorobenzoïque utilisé, on introduit dans ce récipient le mélange réactionnel ayant fini de réagir, on filtre le précipité formé et on le lave à l'eau.

2. Procédé selon la revendication 1, caractérisé en ce que l'on utilise un mélange sulfonitrique contenant 20 à 90 % en masse d'acide sulfurique à 100 % et le complément à 100 % en masse d'acide nitrique à 100 %.

3. Procédé selon les revendications 1 et 2, caractérisé en ce que l'on utilise le mélange sulfonitrique en une quantité telle que la quantité d'acide nitrique qui y est contenu soit de 1 à 1,5 mol par mol d'acide 3-fluorobenzoïque.

4. Procédé selon les revendications 1 à 3, caractérisé en ce que l'on effectue la nitration en la présence supplémentaire d'agents déshydratants.

5. Procédé selon les revendications 1 à 4, caractérisé en ce que l'on dispose dans un récipient 8 à 12 parties en masse d'eau pour 1 partie en masse d'acide 3-fluorobenzoïque utilisé et en ce que l'on maintient la température entre 0 et +100°C lors de l'introduction du mélange réactionnel ayant fini de réagir dans le récipient.

6. Procédé selon les revendications 1 à 5, caractérisé en ce que l'on effectue la filtration à une température comprise entre +10 et +50°C.

7. Procédé selon les revendications 1 à 6, caractérisé en ce que l'on utilise pour le lavage à l'eau une quantité totale de 5 à 10 parties en masse d'eau de lavage pour 1 partie en masse d'acide 3-fluorobenzoïque utilisé, et que l'on effectue le lavage en plusieurs étapes.

8. Procédé selon les revendications 1 à 7, caractérisé en ce que l'on recycle l'eau de lavage dans le récipient dans lequel on introduit le mélange réactionnel ayant fini de réagir.
